# EUROPEAN PATENT APPLICATION

(11) **EP 2 259 202 A2**
(43) Date of publication of application: **08.12.2010**
(21) Application number: 10075379.7
(22) Date of filing: 19.12.2002
(51) Int. Cl.: G06F 19/00

(54) **System and method for operating medical devices**

(30) Priority: 29.01.2002 US 59929
(62) Divisional of application: 02794323.2
(71) Applicant: Baxter International Inc., One Baxter Parkway Deerfield, Illinois 60015-4633 (US)
(72) Inventor: Bui, Tuan, Green Oaks, Illinois 60048 (US); Martucci, James, Libertyville, Illinois 60048 (US); Mihai, Dan, Hanover Park, Illinois 60133 (US)
(74) Representative: Probert, Gareth David

(57) **Abstract**

A system and method for operating medical devices is provided. The system may be used to program a medical device such as an infusion pump. The system may be implemented in a variety of ways including as a computer system. The system may include a first computer at a pharmacy and a second computer at a treatment location. The system sends operating parameters to the medical device after providing various checks to assure that the correct medication is being administered to the correct patient. The system may also include features for confirming the operating parameters are still valid and features for sending alarms to the treatment location if there are discrepancies between the operating parameters, medication identifiers, and/or patient identifiers.

## Description

### Technical Field

This invention relates generally to a system and method for operating medical devices and communication between such devices. More particularly, the present invention relates to a system and method for programming an infusion pump.

### Background of the Invention

Patient care systems typically include computer networks, medical devices for treating a patient, and controls for the medical devices. Although patient care systems have been improved through the use of computerized automation systems and methods, patient care systems continue to rely heavily upon manual data management processes for medical devices and controls for medical devices. For example, nursing stations are typically connected to the computer networks in modem hospitals, but it is still unusual for the computer network to extend to a patient's room. Computer networks offer the opportunity for automated data management processing including the operating and monitoring of medical devices and controls for the medical devices at the point-of-care.

As an example of the current state of the art, U.S. Patent No. 5,781,442 entitled "System and Method for Collecting Data and Managing Patient Care," describes a patient care system with features that include automatic provision of infusion parameters to a pump for configuration of the pump. U.S. Patent No. 5,781,442 is entirely incorporated herein by reference. Despite advances in the field, automated data management technology has been underutilized for point-of-care applications due to a lack of more efficient systems and methods for operating medical devices such as infusion pumps.

### Summary of the Invention

The present invention provides a system and method for operating medical devices. The system and method may be used to program an infusion pump. The system may be implemented in a variety of ways including as a computer program. Briefly described in architecture, the system may be implemented as follows. The system may include a first computer and a second computer for sending operating parameters to the medical device. The first computer may be at a central location such as a pharmacy. The pharmacy computer is designed to accept a first patient identifier and an operating parameter for the medical device. The pharmacy computer may be at a treatment location and is designed to accept a second patient identifier from a first source such as a patient wristband. The pharmacy computer may be a portable digital assistant. The digital assistant is also designed to accept a medication identifier from a medication label, the medication label having been previously attached to a medication container in a pharmacy. The medication identifier includes a third patient identifier. The digital assistant is designed to send the medication identifier to the pharmacy computer if the second patient identifier from the medication label and the third patient identifier from the wristband are equivalent. The patient identifiers are equivalent if there is a sufficient guarantee that they identify the same patient. The pharmacy computer is designed to send the operating parameter directly to the medical device if the third patient identifier is equivalent to the first patient identifier. The system may also include features for confirming the operating parameter is still valid for the patient and features for sending alarms to the treatment location if there are discrepancies between the operating parameters, medication identifiers, and/or patient identifiers.

Other systems, methods, features, and advantages of the present invention will be, or will become, apparent to one having ordinary skill in the art upon examination of the following drawings and detailed description. It is intended that all such additional systems, methods, features, and advantages included within this description, be within the scope of the present invention, and be protected by the accompanying claims.

### Brief Description of the Drawings

The invention can be better understood with reference to the following drawings. The components in the drawings are not necessarily to scale, emphasis instead being placed upon clearly illustrating the principles of the present invention. In the drawings, like reference numerals designate corresponding parts throughout the several views.
FIG. 1 is a graphical representation of a patient care system. The patient care system includes a pharmacy computer, a server, and a digital assistant at a treatment location.
FIG. 2 is a block diagram of a computer system that may be representative of the pharmacy computer, the server, and/or the digital assistant of FIG. 1. The computer system includes a medical device operating system or a portion of the medical device operating system.
FIG. 3 is a flowchart showing a first exemplar embodiment of the medical device operating system of FIG. 2.
FIG. 4 is a flowchart showing a second exemplar embodiment of the medical device operating system of FIG. 2.
FIGS. 5A and 5B depict a flowchart showing a third exemplar embodiment of the medical device operating system of FIG. 2.
FIGS. 6A and 6B depict a flowchart showing a fourth exemplar embodiment of the medical device operating system of FIG. 2.
FIGS. 7A and 7B depict a flowchart showing a fifth exemplar embodiment of the medical device operating system of FIG. 2.
FIGS. 8 depicts a flowchart showing a sixth exemplar embodiment of the medical device operating system of FIG. 2.
FIGS. 9 depicts a flowchart showing a seventh exemplar embodiment of the medical device operating system of FIG. 2.

### Detailed Description

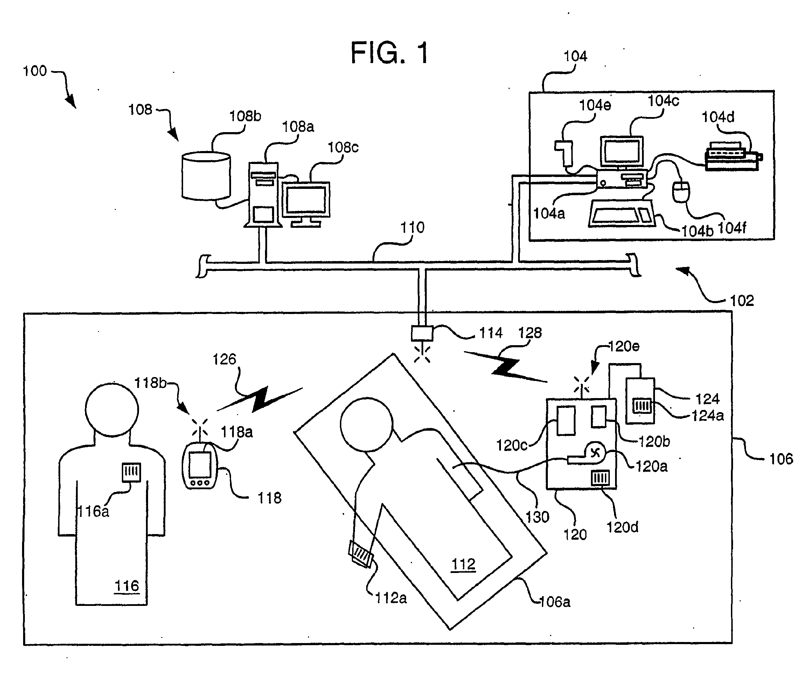

FIG. 1 is a graphical representation of a patient care system 100. The patient care system 100 includes a pharmacy computer 104, a server 108, and a treatment location 106, linked by a network 102. The pharmacy computer 104 may include a processing unit 104a, a keyboard 104b, a video display 104c, a printer 104d, a bar code reader 104e, and a mouse 104f. Although not shown in FIG. 1, the patient care system 100 may also include subsystems for hospital administration, nursing stations, a clinical information subsystem, a hospital information subsystem, and/or other subsystems typically included in patient care systems.

The server 108 may include a central servicing unit 108a, a database 108b, a video display 108c, input/output components, and many other components known to those having ordinary skill in the art. The network 102 includes a cable communication system 110 portion and a wireless communication system portion. The cable communication system 110 may be, but is not limited to, an ethernet cabling system, and a thin net system.

The treatment location 106 may include a treatment bed 106a and an infusion pump 120. In FIG. 1, a care giver 116 and a patient 112 are shown in the treatment location 106. The care giver 116 uses a digital assistant 118 and an infusion pump 120 to administer medication 124 to the patient 112. In the course of treating patient 112, the care giver 116 may use the digital assistant 118 to communicate with the cable communication system 110 of the network 102 via a first wireless communication path 126. The infusion pump 120 may also have the ability to communicate with the cable communication system 110 via a second wireless communication path 128. A wireless transceiver 114 interfaces with the cable communication system 110. The wireless communication system portion of the network may employ technology such as, but not limited to, that known to those having ordinary skill in the art as IEEE 802.11 "Wireless Ethernet," a local area network, wireless local area networks, wireless internet point of presence systems, an Ethernet, the Internet, radio communications, infrared, fiber optic, and telephone. Though shown in FIG. 1 as a wireless communication system, communication paths 126 and 128 may be hardwired communication paths.

In the patient care system 100, a physician (not shown) orders a medication 124 for a patient 112. The medication 124 may be one that is efficient to administer through an infusion pump 120. The order includes information that is sufficient to generate operating parameters for the infusion pump 120. The operating parameters are the information and/or instruction set that is necessary to program a medical device to operate in accordance with the order.

The order is entered in the pharmacy computer 104 via input/output devices such as the keyboard 104b, the mouse 104f, a touch screen display, and/or an electronic physician order entry system. Such input/output devices are known to those having ordinary skill in the art. The processing unit 104a typically transforms a manually entered order into computer readable data. Devices such as the electronic physician order entry system may transform an order into computer readable data prior to introduction to the processing unit 104a. The operating parameters may then be printed in a bar code format by the printer 104d on a medication label 124a in a manner that is known to those having ordinary skill in the art. The medication label 124a may then be affixed to a medication 124 container. The medication 124 container is then transported to the treatment location 106.

At the treatment location, the medication 124 may be mounted on the infusion pump 120 and an intravenous (IV) line 130 may be run from the infusion pump 120 to the patient 112. The infusion pump 120 may include a pumping unit 120a, a keypad 120b, a display 120c, an infusion pump ID 120d, and an antenna 120e.

The patient care system 100 may include a variety of identifiers such as, but not limited to, personnel, equipment, and medication identifiers. In FIG. 1, the care giver 116 may have a care giver badge 116a identifier, the patient 112 may have a wristband 112a identifier, the infusion pump 120 may have an infusion pump ID 120d identifier, and the medication 124 may have a medication label 124a identifier. Care giver badge 116a, wristband 112a, infusion pump ID 120d, and medication label 124a include information to identify the personnel, equipment, or medication they are associated with. The identifiers may also have additional information. For example, the medication label 124a may include information regarding the intended recipient of the medication 124 and operating parameters for infusion pump 120. The information included in the identifiers may be printed, but is preferably in a device readable format such as, but not limited to, an optical readable device format such as a bar code, a radio frequency (RF) device readable format such as an RFID, and/or a laser readable format. The digital assistant 118 may include a display 118a and may have the ability to read the identifiers.

The wristband 112a is typically placed on the patient 112 as the patient 112 enters a medical care facility. The wristband 112a includes a patient identifier. The patient identifier may include printed information to identify the patient and additional information such as a treating physician's name(s). The patient identifier for patient 112 may include information such as, but not limited to, the patient's name, age, social security number, the patient's blood type, address, allergies, a hospital ID number, and the name of a patient's relative.

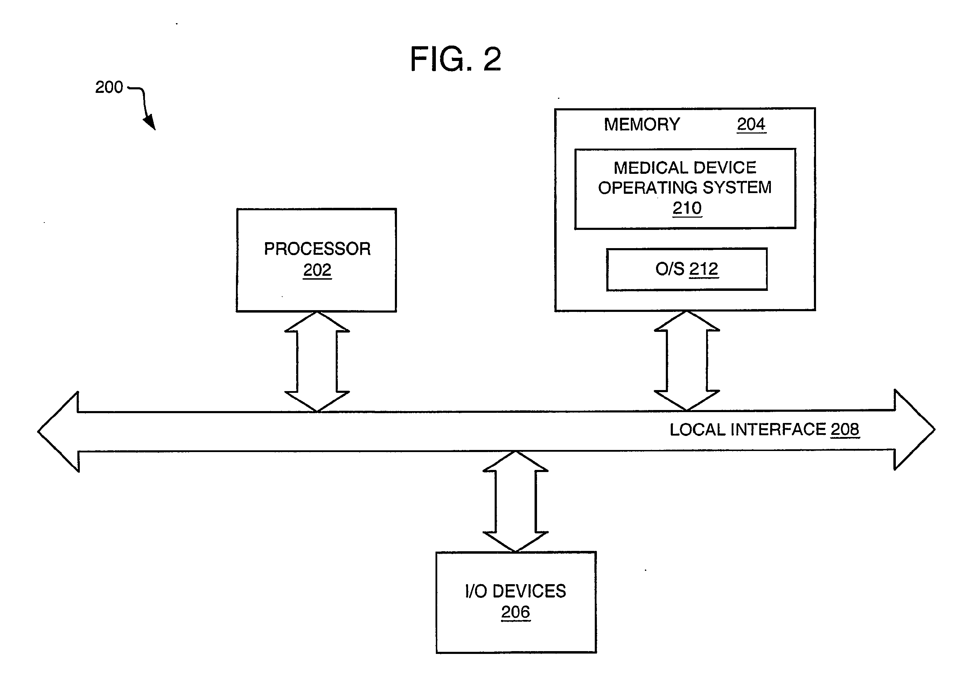

FIG. 2 is a block diagram of a computer 200. Computer 200 may be the pharmacy computer 104, the server 108, the digital assistant 118 of FIG. 1, and/or a computer included in any number of other subsystems that communicate via the network 102. Computer 200 includes a medical device operating system 210. The medical device operating system 210 is used to control the programming of infusion pump 120. In some embodiments, the programming of the infusion pump 120 may be based on operating parameters received from the pharmacy computer 104, and/or another remote computer. In other embodiments, the programming of the infusion pump 120 may be based on operating parameters that are confirmed as correct by the pharmacy computer 104, another remote computer, and/or the care giver 116. The operating parameters and/or confirmations may be transported via the cable communication system 110 and the first and second wireless communication paths 126 and 128.

A critical concern in the art is that the correct medication is administered to the correct patient. Therefore, the medical device operating system 210 includes features to assure the correct medication is administered to the correct patient in an efficient manner. The medical device operating system 210 of the invention can be implemented in software (e.g., firmware), hardware, or a combination thereof. In the currently contemplated best mode, the medical device operating system 210 is implemented in software, as an executable program, and is executed by one or more special or general purpose digital computer(s), such as a personal computer (PC; IBM-compatible, Apple-compatible, or otherwise), personal digital assistant, workstation, minicomputer, or mainframe computer. An example of a general purpose computer that can implement the medical device operating system 210 of the present invention is shown in FIG. 2. The medical device operating system 210 may reside in, or have portions residing in, any computer such as, but not limited to, the pharmacy computer 104, the server 108, and/or the digital assistant 118. Therefore, computer 200 of FIG. 2 may be representative of any computer in which the medical device operating system 210 resides or partially resides.

Generally, in terms of hardware architecture, as shown in FIG. 2, the computer 200 includes a processor 202, memory 204, and one or more input and/or output (I/O) devices 206 (or peripherals) that are communicatively coupled via a local interface 208. The local interface 208 can be, for example, but not limited to, one or more buses or other wired or wireless connections, as is known in the art. The local interface 208 may have additional elements, which are omitted for simplicity, such as controllers, buffers (caches), drivers, repeaters, and receivers, to enable communications. Further, the local interface may include address, control, and/or data connections to enable appropriate communications among the other computer components.

The processor 202 is a hardware device for executing software, particularly software stored in memory 204. The processor 202 can be any custom made or commercially available processor, a central processing unit (CPU), an auxiliary processor among several processors associated with the computer 200, a semiconductor based microprocessor (in the form of a microchip or chip set), a macroprocessor, or generally any device for executing software instructions. Examples of suitable commercially available microprocessors are as follows: a PA-RISC series microprocessor from Hewlett-Packard Company, an 80x86 or Pentium series microprocessor from Intel Corporation, a PowerPC microprocessor from IBM, a Sparc microprocessor from Sun Microsystems, Inc., or a 68xxx series microprocessor from Motorola Corporation.

The memory 204 can include any one or a combination of volatile memory elements (e.g., random access memory (RAM, such as DRAM, SRAM, SDRAM, etc.)) and nonvolatile memory elements (e.g., ROM, hard drive, tape, CDROM, etc.). Moreover, memory 204 may incorporate electronic, magnetic, optical, and/or other types of storage media. The memory 204 can have a distributed architecture where various components are situated remote from one another, but can be accessed by the processor 202.

The software in memory 204 may include one or more separate programs, each of which comprises an ordered listing of executable instructions for implementing logical functions. In the example of FIG. 2, the software in the memory 204 includes the medical device operating system 210 in accordance with the present invention and a suitable operating system (O/S) 212. A non-exhaustive list of examples of suitable commercially available operating systems 212 is as follows: (a) a Windows operating system available from Microsoft Corporation; (b) a Netware operating system available from Novell, Inc.; (c) a Macintosh operating system available from Apple Computer, Inc.; (d) a UNIX operating system, which is available for purchase from many vendors, such as the Hewlett-Packard Company, Sun Microsystems, Inc., and AT&T Corporation; (e) a LINUX operating system, which is freeware that is readily available on the Internet; (f) a run time Vxworks operating system from WindRiver Systems, Inc.; or (g) an appliance-based operating system, such as that implemented in handheld computers or personal digital assistants (PDAs) (e.g., PalmOS available from Palm Computing, Inc., and Windows CE available from Microsoft Corporation). The operating system 212 essentially controls the execution of other computer programs, such as the medical device operating system 210, and provides scheduling, input-output control, file and data management, memory management, and communication control and related services.

The medical device operating system 210 may be a source program, executable program (object code), script, or any other entity comprising a set of instructions to be performed. When a source program, the program needs to be translated via a compiler, assembler, interpreter, or the like, which may or may not be included within the memory 204, so as to operate properly in connection with the O/S 212. Furthermore, the medical device operating system 210 can be written as (a) an object oriented programming language, which has classes of data and methods, or (b) a procedure programming language, which has routines, subroutines, and/or functions, for example but not limited to, C, C++, Pascal, Basic, Fortran, Cobol, Perl, Java, and Ada. In one embodiment, the medical device operating system 210 is written in C++. In other embodiments the medical device operating system is created using Power Builder. The I/O devices 206 may include input devices, for example but not limited to, a keyboard, mouse, scanner, microphone, touch screens, interfaces for various medical devices, bar code readers, stylus, laser readers, radio-frequency device readers, *etc*. Furthermore, the I/O devices 206 may also include output devices, for example but not limited to, a printer, bar code printers, displays, *etc*. Finally, the I/O devices 206 may further include devices that communicate both inputs and outputs, for instance but not limited to, a modulator/demodulator (modem; for accessing another device, system, or network), a radio frequency (RF) or other transceiver, a telephonic interface, a bridge, a router, *etc*.

If the computer 200 is a PC, workstation, PDA, or the like, the software in the memory 204 may further include a basic input output system (BIOS) (not shown in FIG. 2). The BIOS is a set of essential software routines that initialize and test hardware at startup, start the O/S 212, and support the transfer of data among the hardware devices. The BIOS is stored in ROM so that the BIOS can be executed when the computer 200 is activated.

When the computer 200 is in operation, the processor 202 is configured to execute software stored within the memory 204, to communicate data to and from the memory 204, and to generally control operations of the computer 200 pursuant to the software. The medical device operating system 210 and the O/S 212, in whole or in part, but typically the latter, are read by the processor 202, perhaps buffered within the processor 202, and then executed.

When the medical device operating system 210 is implemented in software, as is shown in FIG. 2, it should be noted that the medical device operating system 210 can be stored on any computer readable medium for use by or in connection with any computer related system or method. In the context of this document, a computer readable medium is an electronic, magnetic, optical, or other physical device or means that can contain or store a computer program for use by or in connection with a computer related system or method. The medical device operating system 210 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be for example, but not limited to, an electronic, magnetic, optical, electromagnetic, infrared, or semiconductor system, apparatus, device, or propagation medium. More specific examples (a non-exhaustive list) of the computer-readable medium would include the following: an electrical connection (electronic) having one or more wires, a portable computer diskette (magnetic), a random access memory (RAM) (electronic), a read-only memory (ROM) (electronic), an erasable programmable read-only memory (EPROM, EEPROM, or Flash memory) (electronic), an optical fiber (optical), and a portable compact disc read-only memory (CDROM) (optical). Note that the computer-readable medium could even be paper or another suitable medium upon which the program is printed, as the program can be electronically captured, via, for instance, optical scanning of the paper or other medium, then compiled, interpreted or otherwise processed in a suitable manner if necessary, and then stored in a computer memory.

In another embodiment, where the medical device operating system 210 is implemented in hardware, the medical device operating system 210 can be implemented with any or a combination of the following technologies, which are each well known in the art: a discrete logic circuit(s) having logic gates for implementing logic functions upon data signals, an application specific integrated circuit (ASIC) having appropriate combinational logic gates, a programmable gate array(s) (PGA), a field programmable gate array (FPGA), *etc.*

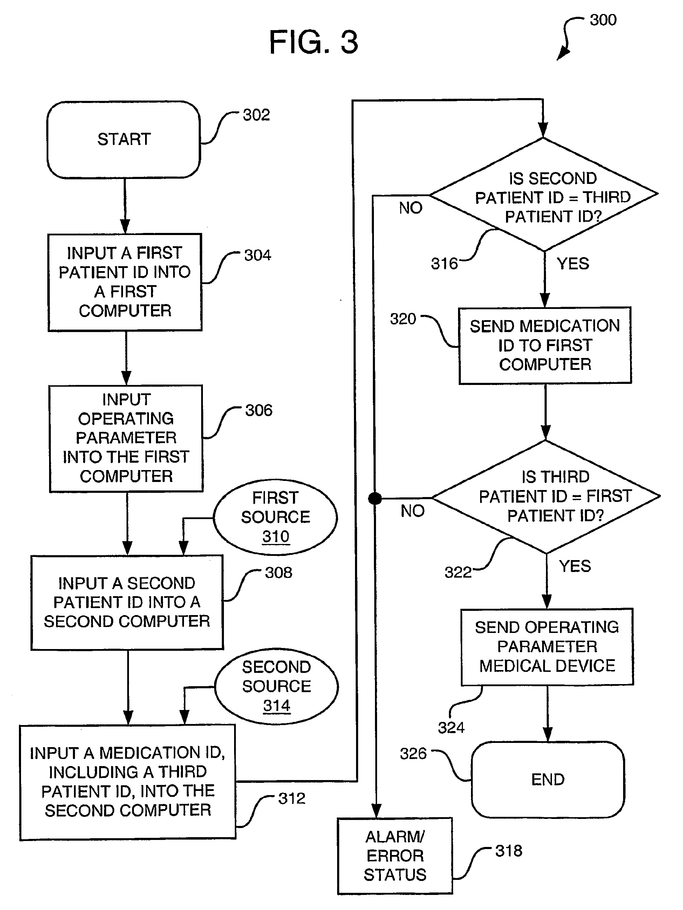

Any process descriptions or blocks in figures, such as FIGS. 3-9B, should be understood as representing modules, segments, or portions of code which include one or more executable instructions for implementing specific logical functions or steps in the process, and alternate implementations are included within the scope of the embodiments of the present invention in which functions may be executed out of order from that shown or discussed, including substantially concurrently or in reverse order, depending on the functionality involved, as would be understood by those having ordinary skill in the art.

FIG. 3 is a flowchart showing first exemplar embodiment 300 of the medical device operating system 210 of FIG. 2. The medical device operating system 300 is called in block 302. After the medical device operating system 300 is called in block 302, the system 210 moves to block 304. In block 304, a first computer, such as the pharmacy computer 104, accepts a first patient identifier (ID). Though not limited to these examples, the first computer may also be the server 108, and/or a computer at a central location such as a nursing station, a clinical information subsystem, and/or a hospital information system. The first patient ID may be derived from input sources such as, but not limited to, admission records, orders, an electronic physician order entry system, and/or prescriptions. Block 304 may include converting a signal generated by an input device, such as a keyboard and/or bar code reader, to a computer readable medium format. After block 304, the system 300 goes to block 306.

Throughout this document and the related claims, "central location" and "remote location" are relative terms to each other. A "remote location" is any location where a patient is receiving treatment through a controlled medical device, such as a patient treatment location 106 where patient 112 is receiving treatment through an infusion pump 120. "Central location" is any location, other than the remote location, where parameters for operating the medical device are accessible such as, but not limited to, the location of the pharmacy computer 104 and the server 108. In a typical arrangement, several remote locations, such as treatment location 106, are in communication with a central location.

In block 306, the first computer, for example pharmacy computer 104, accepts an operating parameter (O.P.). The operating parameter may be a parameter such as, but not limited to, a flow rate per unit of time, a quantity of medication, a dosing unit, a dosing duration, a dosing volume, a drug name, a dose unit, and a monitoring limit. After block 306, the system 300 goes to block 308.

In block 308, a second computer, for example the digital assistant 118, accepts a second patient identifier from a first source 310 such as wristband 112a. The second computer may also be another computer located at a remote location. First source 30 may be a variety of other sources such as, but not limited to, a bar code such as a bar code included in wristband 112a, a bar code reader, a tag, a drug label, laser readable data, and radio-frequency readable data. Block 308 may include converting a signal generated by an input device, such as a bar code reader associated with digital assistant 118, to a computer readable medium format. After block 308, the system 300 goes to block 312.

In block 312, the second computer, for example the digital assistant 118, accepts a medication identifier (ID) from a second source 314. The medication ID includes a third patient ID. The second source 314 may be medication label 124a. The medication ID may be an identifier such as, but not limited to, a drug name, a dosage, a manufacturer, a batch, an expiration date, and/or a drug prescriber. After block 310, the system 300 goes to block 316.

In block 316, the system 300 determines whether the second patient ID of block 308 is equivalent to the third patient ID of block 312. The determination will often be made by the device that gathers data from the first and second sources 310 and 314. For example, a care giver 116 may use the digital assistant 118 to read a bar code from a patient's wristband 112a. The care giver 116 may then use the digital assistant 118 to read medication label 124a. The digital assistant 118 may then determine whether the second patient ID from the patient's wristband is equivalent to the third patient ID of from the medication label 124a. Two identifiers are equivalent if they are similar enough to assure that they both identify the same person, device, or medication. The system 300 may require that identifiers are identical to each, or the system 300 may allow some flexibility to allow for a determination of equivalence to be made even though the identifiers are not identical, if the identifiers match to a degree to assure the identifiers are referring to the same person, device, or medication.

If the system 300 determines the second patient ID of block 308 is not equivalent to the third patient ID of block 312, the system 300 moves to block 318 where an alarm/error status is provided by the system 300. If the system 300 determines the second patient ID of block 308 is equivalent to the third patient ID of block 312, the system 300 moves to block 320. In block 320, the system 300 sends the medication ID of block 312 to the first computer. Under the scenario described above, the digital assistant 118 sends the medication ID to the pharmacy computer 104. After block 320, the system 300 goes to block 322.

In block 322, the system 300 determines whether the third patient ID of block 312 is equivalent to the first patient ID of block 304. The determination will often be made by the first computer, for example, the pharmacy computer 104a. If the system 300 determines the third patient ID of block 312 is not equivalent to the first patient ID of block 304, the system 300 moves to block 318. If the system 300 determines the third patient ID of block 312 is equivalent to the first patient ID of block 304, the system 300 moves to block 324. Since the second and third patient IDs have already been determined to be equivalent in block 316, the system 300 may also be viewed as determining in block 322 whether to go to block 318 or block 324 based on whether the second patient ID of block 312 is equivalent to the first patient ID of block 304.

In block 324, the system 300 sends the operating parameters of block 306 to a medical device such as infusion pump 120. After block 324, the system 300 moves to block 326 where the system 300 terminates.

One benefit of the medical device operating system 210 is that the operating parameters for the medical device do not have to pass through digital assistant 118, or any other computer in the remote location prior to the operating parameters being available to program the medical device. Bypassing computers at the remote location eliminates a potential source of errors in administering medication 124 to a patient 112. The operating parameters for the medical device may be sent "directly" to the medical device. In this context, "directly" meaning that the operating parameters may be sent to the medical device without passing through the digital assistant, or any other computer in the remote location.

In another embodiment, the system 300 may include an additional block (not shown) where the first computer accepts a second medication ID. The second medication ID may be entered at the time the first computer receives the first patient ID and the operating parameter or the second medication ID may be a revised first medication ID. For example, the second medication ID may be part of the prescription or electronic physician order entry that is the source for the first patient ID and the operating parameters. The system 300 may then confirm the first and second medication IDs are equivalent prior to sending the operating parameters to the medical device. The second medication ID may be replaced by a revised first medication ID between the time the prescription is entered and the time the medication 124 arrives at the treatment location 106. The system 300 will then sound an alarm if second medication ID is not equivalent to the first medication ID that was included in the medication label 124a.

In a further embodiment, the system 300 may include an additional block (not shown) where the operating parameter is used to program the medical device.

In one implementation of system 300, an order is entered in pharmacy computer 104. The order includes a first patient identifier and an operating parameter. The pharmacy computer 104 generates a medication label 124a that is affixed to medication 124. The medication 124 is sent to a treatment location 106. At treatment location 106, a care giver 116 reads a patient's wristband 112a and the medication label 124a with a digital assistant 118. The digital assistant 118 determines whether patient identifiers associated with the medication label 124a and the wristband 112a identify the same patient 112. The system 300 then sends the medication identifier to the pharmacy computer 104. The pharmacy computer 104 confirms the medication label 124a identifies the same patient as the order and sends the operating parameter to an infusion pump. The operating parameter may be sent directly to the infusion pump. The operating parameter is then used to program the infusion pump to administer the medication 124 to the patient 112.

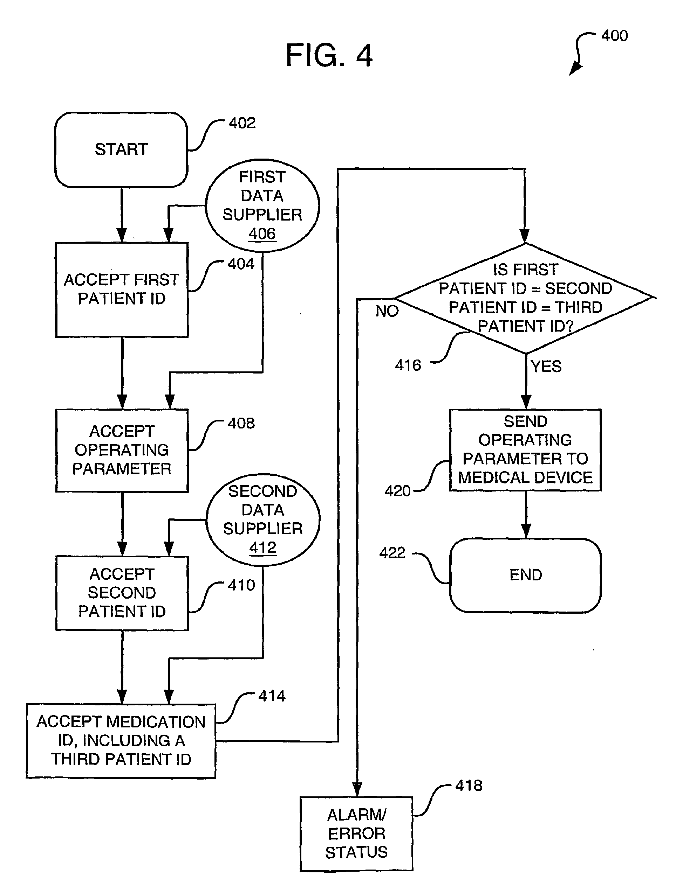

FIG. 4 is a flowchart showing a second exemplar embodiment 400 of the medical device operating system 210 of FIG. 2. The medical device operating system 400 is called in block 402. After the medical device operating system 400 is called in block 402, the system 400 moves to block 404. In block 404, the system 400 accepts a first input at a first computer. The first input comes from a first data supplier 406, and the first input includes a first patient identifier (ID) and an operating parameter.

The system 400 may be stored in the memory of the first computer. The first data supplier 406 may be one or more input devices for the first computer. After block 404, the system 400 goes to block 408. In block 408, the system 400 accepts the operating parameter (O.P.) of the first input at the first computer. The first data supplier 406 may also supply the operating parameter. After block 408, the system 400 goes to block 410.

In block 410, the system 400 accepts a second input, including a second patient identifier, from a second data supplier 412 such as the digital assistant 118. The second data supplier 412 may also be a second computer such as the server 108, or another computer located at a remote location. The data supplied by second data supplier 412 may be based on information derived from a device such as wristband 112a attached to a patient 112. The device may also be another device that includes information in a machine readable format such as, but not limited to, a bar code, a bar code reader, a tag, a drug label, a laser readable format, a camera-type bar code format, an RFID format, a magnetic stripe, and a radio-frequency readable format. Block 410 may include converting a signal generated by an input device, such as a bar code reader associated with digital assistant 118, to a computer readable medium format. After block 410, the system 400 goes to block 414.

In block 414, the second computer accepts a third input, including a medication identifier, from the second data supplier 412. The medication identifier includes a third patient ID. The medication identifier may be based on information derived from a medication label 124a. After block 414, the system 400 goes to block 416.

In block 416, the system 400 determines whether the first patient identifier of block 404 is equivalent to the second patient ID of block 410 and to the third patient ID of block 414. If the system 400 determines the patient identifiers are not equivalent, the system 400 moves to block 418 where an alarm/error status is provided by the system 400. If the system 400 determines the patient identifiers are equivalent, the system 400 moves to block 420. In block 420, the system 400 sends the operating parameter of block 408 to a medical device. After block 420, the system 400 moves to block 422 where the system 400 terminates.

In another embodiment, the system 400 may include an additional block (not shown) where the first computer accepts a second medication identifier. In this embodiment, the system 400 would only send the operating parameters to the medical device if the first and second medication identifiers are equivalent.

In a further embodiment, the system 400 may include an additional block (not shown) where the operating parameter is used to program the medical device.

In one implementation of system 400, an order is entered in a pharmacy computer 104. The order includes a first patient identifier and an operating parameter. The pharmacy computer 104 generates a medication label 124a that is affixed to medication 124. The medication 124 is sent to a treatment location 106. At the treatment location 106, a care giver 116 reads a patient's wristband 112a and the medication label 124a with a digital assistant 118. The pharmacy computer 104 then confirms the order, the wristband 112a, and the medication label 124a all identify the same patient 112. The system 400 then sends the operating parameter from the pharmacy computer 104 directly to an infusion pump. The operating parameter is then used to program the infusion pump to administer the medication 124 to the patient 112.

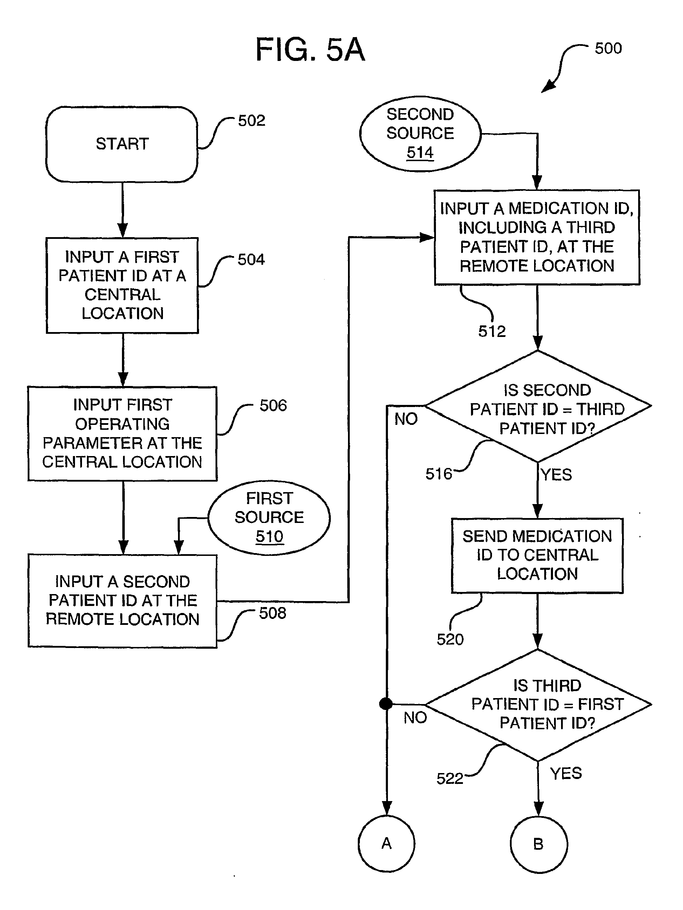

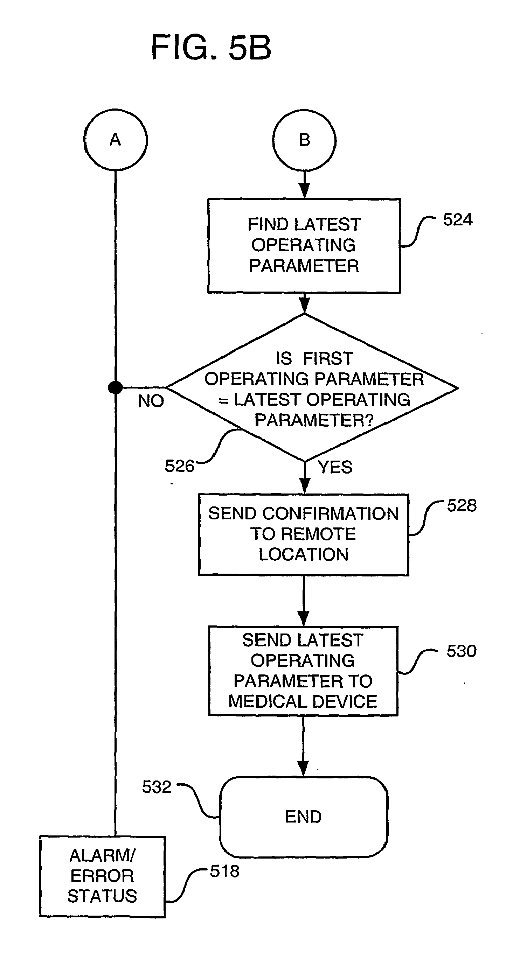

FIGS. 5A and 5B depict a flowchart showing a third exemplar embodiment 500 of the medical device operating system 210 of FIG. 2. The medical device operating system 500 is called in block 502. After the system 500 is called in block 502, the system 500 moves to block 504. In block 504, a first patient identifier (ID) is input at a central location. The input may be to a computer such as, but not limited to, the pharmacy computer 104a, the server 108, and/or a computer at a central location such as a nursing station, a clinical information subsystem, and/or a hospital information system. The first patient ID may be derived from input sources such as, but not limited to, admission records, orders, an electronic physician order entry system and/or prescriptions. After block 504, the system 500 goes to block 506.

In block 506, a first operating parameter is input at the central location. After block 506, the system 500 goes to block 508. In block 508, a second patient identifier from a first source 510 is input at the remote location. The input may be to a computer such as the digital assistant 118 or another computer located at the remote location. First source 510 may be a variety of sources such as wristband 112a. After block 508, the system 500 goes to block 512.

In block 512, a medication identifier (ID) from a second source 514, such as a medication label 124a, is input at the remote location. The input may again be to a computer such as the digital assistant 118 or another computer located at a remote location. The medication ID includes a third patient ID. After block 512, the system 500 goes to block 516.

In block 516, the system 500 determines whether the second patient ID of block 508 is equivalent to the third patient ID of block 512. The determination may be made by the remote computer. If the system 500 determines the second patient ID is not equivalent to the third patient ID, the system 500 moves to block 518 where an alarm/error status is provided by the system 500. If the system 500 determines the second patient ID is equivalent to the third patient ID, the system 500 moves to block 520. In block 520, the system 500 sends the medication ID of block 512 to the central location. After block 520, the system 500 goes to block 522.

In block 522, the system 500 determines whether the third patient ID of block 512 is equivalent to the first patient ID of block 504. The determination will often be made by a computer at the central location. If the system 500 determines the third patient ID is not equivalent to the first patient ID, the system 500 moves to block 518. If the system 500 determines the third patient ID is equivalent to the first patient ID, the system 500 moves to block 524. Since the second and third patient IDs have already been determined to be equivalent in block 516, the system 500 may also be viewed as determining in block 522 whether to go to block 518 or block 524 based on whether the second patient ID of block 508 is equivalent to the first patient ID of block 504.

In block 524, the system 500 searches for the latest operating parameter related to the patient. Physicians or other treatment providers often change prescribed medications and/or operating parameters for medical devices. For example, in the morning a physician may prescribe a medication to be administered in the afternoon according to an operating parameter for a medical device. Prior to the time the prescription is administered, the physician may receive new information causing the physician to change the medication and/or the operating parameter. In block 524, the system 500 searches for the most recent operating parameter. After block 524, the system goes to block 526.

In block 526, the system 500 determines whether the first operating parameter of block 506 is equivalent to the latest operating parameter of block 524. If the system 500 determines the first operating parameter is not equivalent to the latest operating parameter, the system 500 goes to block 518. If the system 500 determines the first operating parameter is equivalent to the latest operating parameter, the system 500 goes to block 528.

In block 528, a confirmation is sent to the remote location. The confirmation may be sent the digital assistant 118 so that the care giver 116 is informed that the operating parameter is to be sent to the medical device. After block 528, the system goes to block 530. In block 530, the system 500 sends the latest operating parameter to the medical device such as the infusion pump 120. Since the first and latest operating parameter has already been determined to be equivalent in block 526, the system 500 may also be viewed as sending the first operating parameter to the medical device. After block 530, the system 500 moves to block 532 where the system 500 terminates. In another embodiment, an additional block (not shown) is included where the operating parameter is used to program the medical device.

In one implementation of system 500, an order is entered in a pharmacy computer 104. The order includes a first patient identifier and a plurality of operating parameters. The pharmacy computer 104 generates a medication label 124a that is affixed to medication 124. The medication 124 is sent to a treatment location 106. The care giver 116 then reads a patient's wristband 112a and the medication label 124a with a digital assistant 118. The digital assistant 118 determines the patient identifiers associated with the medication label 124a and the wristband 112a identify the same patient 112. The system 500 then sends the medication identifier to the pharmacy computer 104. The pharmacy computer 104 confirms the medication label 124a identifies the same patient as the order. The system then searches the pharmacy computer 104 to determine if a new operating parameter has been entered for the patient 112. The pharmacy computer then determines whether the latest and the first operating parameter are equivalent and sends a confirmation to the digital assistant 118. The system 500 then sends the operating parameter to the infusion pump. The operating parameter is then used to program the infusion pump to administer the medication 124 to the patient 112.

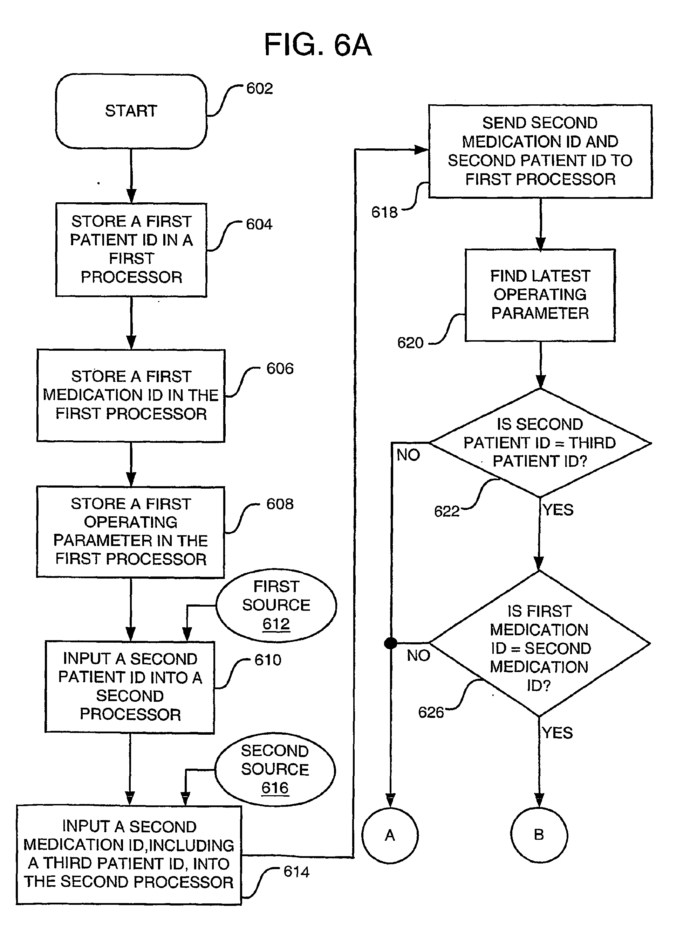

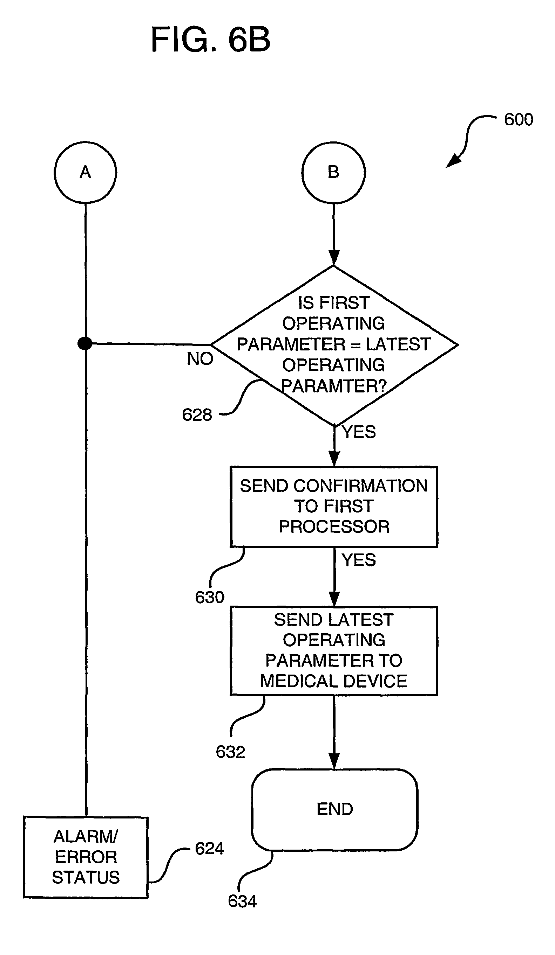

FIGS. 6A and 6B depict a flowchart showing a fourth exemplar embodiment 600 of the medical device operating system 210 of FIG. 2. The medical device operating system 600 is called in block 602. After the system 600 is called in block 602, the system 600 moves to block 604. In block 604, a first patient identifier (ID) is stored in a first processor. The processor may be included in a computer such as, but not limited to, the pharmacy computer 104a, the server 108, and/or a computer at a central location such as a nursing station, a clinical information subsystem, and/or a hospital information system. The first patient ID may be derived from input sources such as, but not limited to, admission records, orders, an electronic physician order entry system and/or prescriptions. After block 604, the system 600 goes to block 606. In block 606, a first operating parameter is stored in the first processor. The first operating parameter may be derived from an electronic physician order entry system and/or prescriptions. After block 608, the system 600 goes to block 610.

In block 610, a second patient identifier from a first source 612 is input into a second processor. The second processor may be at a remote location. The second processor may be included in a digital assistant 118. The input of block 612 may be via a digital assistant input device such as a barcode reader. First source 610 may be a variety of sources such as wristband 112a. After block 610, the system 600 goes to block 614.

In block 614, a second medication identifier (ID) from a second source 616, such as a medication label 124a, is input into the second processor. The second medication ID includes a third patient ID. After block 614, the system 600 goes to block 618. In block 618, the second medication identifier of block 614 and the second patient identifier of block 610 are sent to the first processor. After block 618, the system 600 goes to block 620. In block 620, the system searches for and finds the latest operating parameter. After block 620, the system 600 goes to block 622.

In block 622, the system 600 determines whether the second patient ID of block 610 is equivalent to the third patient ID of block 614. The determination may be made by the first processor. If the system 600 determines the second patient ID is not equivalent to the third patient ID, the system 600 moves to block 624 where an alarm/error status is provided by the system 600. If the system 600 determines the second patient ID is equivalent to the third patient ID, the system 600 moves to block 626.

In block 626, the system 600 determines whether the first medication identifier of block 606 is equivalent to the second medication identifier of block 614. The determination will often be made by the first processor. If the system 600 determines the first medication identifier is not equivalent to the second medication identifier, the system 600 moves to block 624. If the system 600 determines the first medication identifier is equivalent to the second medication identifier, the system 600 moves to block 628.

In block 628, the system 600 determines whether the first operating parameter of block 608 is equivalent to the latest operating parameter of block 620. If the system 600 determines the first operating parameter is not equivalent to the latest operating parameter, the system 600 goes to block 624. If the system 600 determines the first operating parameter is equivalent to the latest operating parameter, the system 600 goes to block 630.

In block 630, a confirmation is sent to the second processor. After block 630, the system 600 goes to block 632. In block 632, the system 600 sends the latest operating parameter to the medical device such as the infusion pump 120. Since the first and latest operating parameters have already been determined to be equivalent in block 628, the system 600 may also be viewed as sending the first operating parameter to the medical device. After block 632, the system 600 moves to block 634 where the system 600 terminates. In another embodiment, an additional block (not shown) is included where the operating parameter is used to program the medical device.

In one implementation of system 600, an order is entered in a pharmacy computer 104. The order includes a first patient identifier, a first medication identifier, and an operating parameter. The pharmacy computer 104 generates a medication label 124a that is affixed to medication 124. The medication 124 is sent to a treatment location 106. The care giver 116 then reads a second patient identifier from a patient's wristband 112a and a second medication identifier from the medication label 124a using a digital assistant 118. The second patient identifier and the second medication identifier are then sent to the pharmacy computer. The pharmacy computer 104 determines whether the patient identifiers associated with the medication label 124a and the wristband 112a identify the same patient 112. The pharmacy computer then determines whether the first medication identifier entered in the pharmacy computer 104 identifies the same medication as the medication identifier associated with the medication label 124a. The pharmacy computer 104 then determines whether the latest and the first operating parameter are equivalent and, if they are equivalent, sends a confirmation to the digital assistant 118. The system 600 then sends the operating parameter to the infusion pump 120. The operating parameter is then used to program the infusion pump 120 to administer the medication 124 to the patient 112.

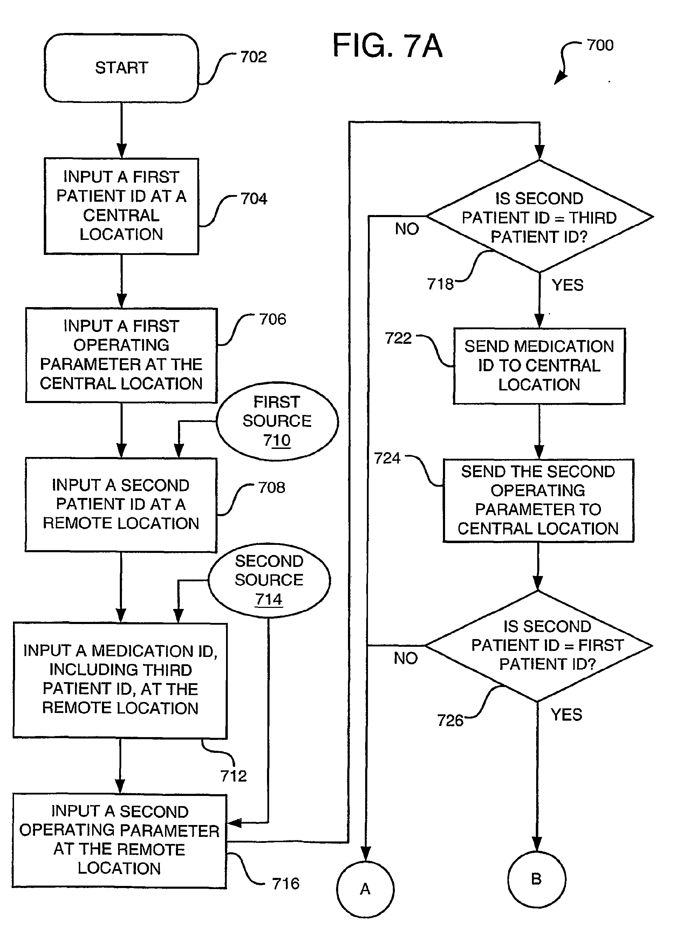

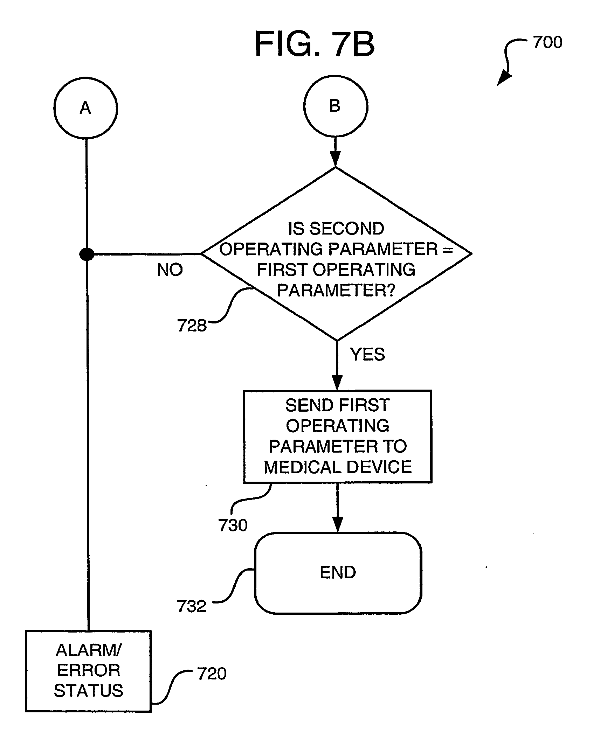

FIGS. 7A and 7B depict a flowchart showing a fifth exemplar embodiment 700 of the medical device operating system 210 of FIG. 2. The medical device operating system 700 is called in block 702. After the system 700 is called in block 702, the system 700 moves to block 704. In block 704, a first patient identifier (ID) is stored at a central location such as the pharmacy. The first patient ID may be derived from input sources such as, but not limited to, admission records, orders, an electronic physician order entry system and/or prescriptions. After block 704, the system 700 goes to block 706. In block 706, a first operating parameter is stored at the central location. The first operating parameter may be derived from an electronic physician order entry system and/or prescriptions. After block 706, the system 700 goes to block 708.

In block 708, a second patient identifier from a first source 710 is input at a remote location. The first source 710 may be wristband 112a. A bar code reader that is integral with the medical device may be used to input information from the wristband 112a to a processor that is also integral with the medical device. First source 710 may also be viewed as the wristband 112a. After block 708, the system 700 goes to block 712.

In block 712, a medication identifier (ID) from a second source 714, such as a medication label 124a, is input at the remote location. The second medication ID includes a third patient ID. The second source 714 may be a medication label 124a that is also read using the barcode reader that is integral with the medical device. After block 712, the system 700 goes to block 716. In block 716, a second operating parameter from the second source, such as the medication label 124a, is input at the remote location. After block 716, the system 700 goes to block 718.

In block 718, the system 700 determines whether the second patient ID of block 708 is equivalent to the third patient ID of block 712. The determination may be made by a processor that is integral with the medical device. If the system 700 determines the second patient ID is not equivalent to the third patient ID, the system 700 moves to block 720 where an alarm/error status is provided by the system 700. If the system 700 determines the second patient ID is equivalent to the third patient ID, the system 700 moves to block 722.

In block 722, the system 700 sends the medication identifier of block 712 to the central location. After block 722, the system goes to block 724. In block 724, the system 700 sends the second operating parameter to the central location. After block 724, the system 700 goes to block 726.

In block 726, the system 700 determines whether the third patient ID of block 712 is equivalent to the first patient ID of block 704. The determination may be made by the pharmacy computer 104. If the system 700 determines the third patient ID is not equivalent to the first patient ID, the system 700 moves to block 720 where the alarm/error status is provided by the system 700. If the system 700 determines the third patient ID is equivalent to the first patient ID, the system 700 moves to block 728.

In block 728, the system 700 determines whether the second operating parameter of block 716 is equivalent to the first operating parameter of block 706. If the system 700 determines the second operating parameter is not equivalent to the first operating parameter, the system 700 goes to block 720. If the system 700 determines the second operating parameter is equivalent to the first operating parameter, the system 700 goes to block 730.

In block 730, the system 700 sends the first operating parameter to the medical device such as the infusion pump 120. Since the first and second operating parameters have already been determined to be equivalent in block 728, the system 700 may also be viewed as sending the second operating parameter to the medical device. After block 730, the system 700 moves to block 732 where the system 700 terminates. In another embodiment, an additional block (not shown) is included where the operating parameter is used to program the medical device.

In one implementation of system 700, an order is entered in a pharmacy computer 104. The order includes a first patient identifier and an operating parameter. The pharmacy computer 104 generates a medication label 124a that is affixed to medication 124. The medication 124 is sent to a treatment location 106. The care giver 116 then reads a second patient identifier from a patient's wristband 112a and a medication identifier from the medication label 124a using a bar code reader that is integral with the medical device. The medication label 124a also provides a second operating parameter. A processor that is integral with the medical device then determines whether the patient identifiers associated with the medication label 124a and the wristband 112a identify the same patient 112. The medication identifier and the second operating parameter are then sent to the pharmacy computer. The pharmacy computer 104 then determines whether the medication identifier identifies the same patient. The pharmacy computer 104 then determines whether the first and second operating parameters are equivalent and, if they are equivalent, sends the operating parameter to the infusion pump 120. The operating parameter is then used to program the infusion pump 120 to administer the medication 124 to the patient 112.

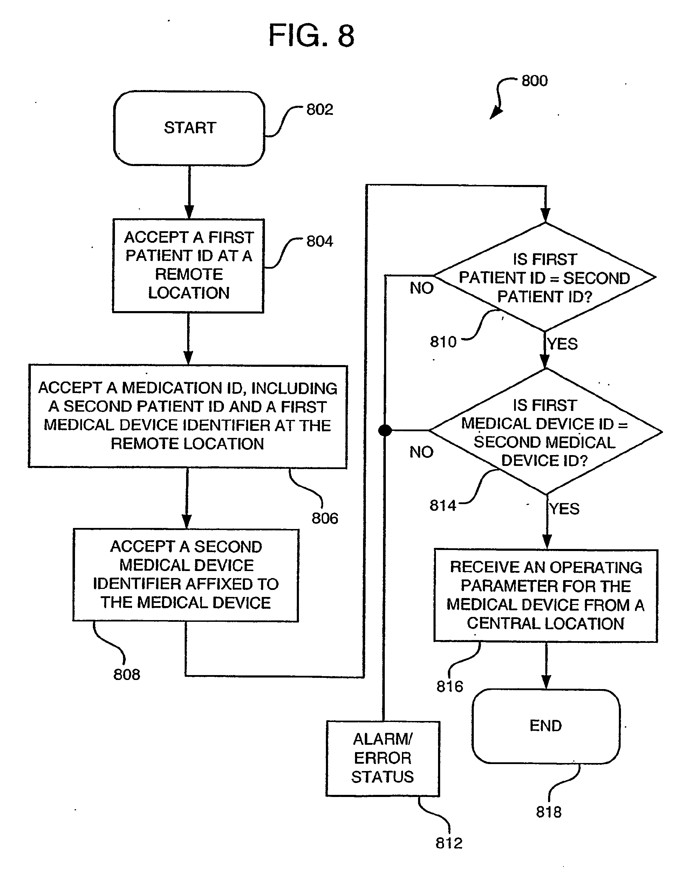

FIG. 8 is a flowchart showing a sixth exemplar embodiment 800 of the medical device operating system 210 of FIG. 2. The medical device operating system 800 is called in block 802. After the system 800 is called in block 802, the system 800 moves to block 804. In block 804, the system accepts a first patient identifier (ID) at a remote location. The first patient identifier may be derived from wristband 112a. After block 804, the system 800 moves to block 806.

In block 806, the system 800 accepts a medication identifier (ID). The medication identifier may be derived from a medication label 124a. The medication identifier includes a second patient identifier and a first medical device identifier. The medical device identifier may indicate a unique medical device, such as an infusion pump, or the medical device identifier may indicate a particular model of a medical device. After block 806, the system 800 goes to block 808.

In block 808, the system accepts a second medical device identifier. The second medical device identifier may be derived from a label, such as infusion pump ID 120d, that is affixed to a medical device. After block 808, the system 800 goes to block 810.

In block 810, the system 800 determines whether the first patient identifier of block 804 is equivalent to the second patient identifier of block 806. If the first patient identifier is not equivalent to the second patient identifier, the system goes to block 812 where an alarm/error status is provided. If the first patient identifier is equivalent to the second patient identifier, the system goes to block 814.

In block 814, the system 800 determines whether the first medical device identifier of block 806 is equivalent to the second medical device identifier of block 808. If the first medical device identifier is not equivalent to the second medical device identifier, the system goes to block 812. If the first medical device identifier is equivalent to the second medical device identifier, the system goes to block 816.

In block 816, the system 800 receives an operating parameter for the medical device. The medical device receives the operating parameter from a central location. After block 816, the system 800 goes to block 818 where the system 800 terminates.

In one implementation of system 800, a care giver 116 reads a first patient identifier from a patient's wristband 112a and a medication identifier from the medication label 124a using a digital assistant 118 having a bar code reader. The medication identifier includes a second patient identifier and a medical device identifier. The medical device identifier may uniquely identify one infusion pump 120 in the patient care system 100. The care giver 116 then reads a second medical identifier that is affixed to a medical device. The second medical identifier may also uniquely identify one infusion pump 120 in the patient care system 100. The digital assistant may then determine whether the first and second patient identifiers identify the same patient. If the first and second patient identifiers identify the same patient, the system 800 then determines whether the first and second medical device identifiers identify the same medical device. If the first and second medical identifiers are associated with the same medical device, the system 800 receives an operating parameter for the medical device from the pharmacy computer 104. System 800 is particularly useful when there are several similar medical devices in the same treatment location. Through system 800 several medical devices that are administering medication to the same patient may be controlled.

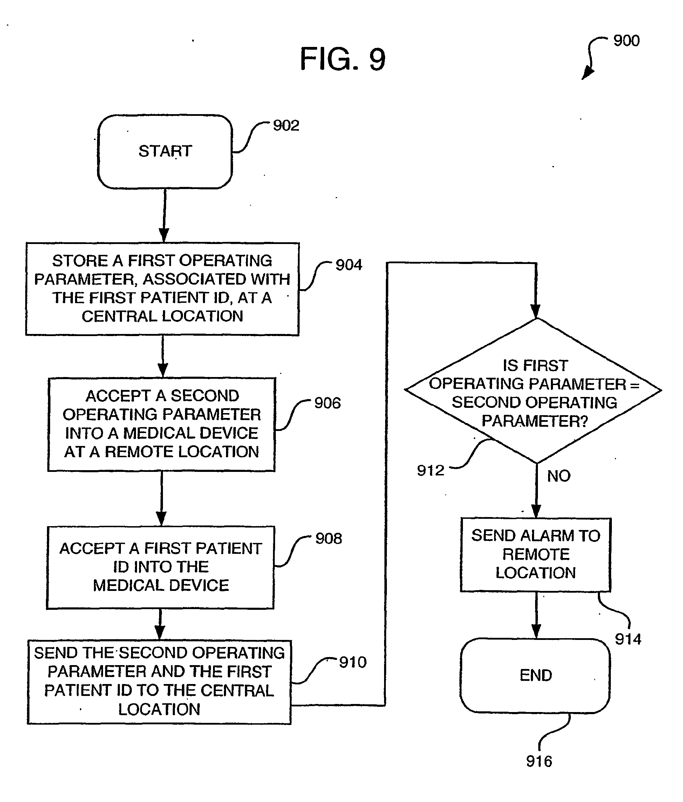

FIG. 9 is a flowchart showing a seventh exemplar embodiment 900 of the medical device operating system 210 of FIG. 2. The medical device operating system 900 is called in block 902. After the system 900 is called in block 902, the system 900 moves to block 904. In block 904, a first operating parameter is stored at a central location, such as the pharmacy computer 104. The first operating parameter is associated with a first patient identifier. After block 904, the system 900 goes to block 906.

In block 906, the medical device accepts a second operating parameter. The second operating parameter may be entered manually through a keypad of the medical device, such as keypad 120b of infusion pump 120. After block 906, the system 900 moves to block 908. In block 908, the medical device accepts the first patient identifier. The first patient identifier may also be entered manually through the keypad. The first operating parameter and the first patient identifier of system 900 may be derived from a medication label 124a. After block 908, the system 900 moves to block 910.

In block 910, the system 900 sends the second operating parameter and the first patient identifier to the central location. In block 912, the system 900 determines whether the first operating parameter is equivalent to the second operating parameter. If the first operating parameter is not equivalent to the second operating parameter, the system 900 goes to block 914. In block 914, the system sends an alarm to the remote location. After block 914, the system goes to block 916, where the system 900 terminates. In an additional embodiment, the alarm of system 900 may be triggered if a time limit is exceeded between the storage of the first operating parameter in block 904 and the sending of the second operating parameter of block 910.

In one implementation of system 900, an order is entered in a pharmacy computer 104. The order includes a first operating parameter and a patient identifier associated with the first operating parameter. The pharmacy computer 104 generates a medication label 124a that is affixed to medication 124. The medication 124 is sent to a treatment location 106. The care giver 116 then reads the second operating parameter and the patient identifier from the medication label 124a and enters the second operating parameter and the patient identifier into the infusion pump 120 using the keypad 120d. The system 900 then sends the second operating parameter and the patient identifier to the pharmacy computer 104. The pharmacy computer 104 then compares the first and second operating parameters and sends an alarm to the medical device if the first and second operating parameters are not equivalent. The system may also send an alarm if a time limit is exceeded between the time the first operating parameter is entered in the pharmacy computer 104 and the time the second operating parameter is sent from the infusion pump to the pharmacy computer 104.

It should be emphasized that the above-described embodiments of the present invention, particularly, any "preferred" embodiments, are possible examples of implementations, merely set forth for a clear understanding of the principles of the invention. Many variations and modifications may be made to the above-described embodiment(s) of the invention without substantially departing from the spirit and principles of the invention. All such modifications are intended to be included herein within the scope of this disclosure and the present invention and protected by the following claims.

Preferred aspects of the present invention are set out in the following numbered paragraphs of the description:
1. A system for operating an infusion pump, the system comprising:
   a first computer at a central location, the first computer designed to accept a first patient identifier and an operating parameter for the infusion pump;
   a second computer at a remote location, the second computer designed to accept a second patent identifier from a first source, the second computer designed to accept a medication identifier from a second source, the medication identifier including a third patient identifier,
   where the second computer is designed to send the medication identifier to the first computer if the second patient identifier and the third patient identifier are equivalent;
   where the first computer is designed to send the operating parameter to the infusion pump if the third patient identifier is equivalent to the first patient identifier, where the operating parameter does not pass through the second computer.
2. The system of paragraph 1, where the first computer is designed to accept a second medication identifier, where the first computer is designed to send the operating parameter to the infusion pump only if the first medication identifier is equivalent to the second medication identifier.
3. The system of paragraph 1, where the first patient identifier is one of a group of identifiers, where the group of identifiers consists of: a patient name, a patient social security number, a patient blood type, a patient address, a patient's allergy, and a name of a patient's relative.
4. The system of paragraph 1, where the operating parameter is one of a group of operating parameters, where the group of operating parameters consists of a medication flow per unit of time, a quantity of medication, a dosing unit, a dosing duration, a dosing volume, a drug name, a dose unit, and a monitoring limit.
5. The system of paragraph 1, where the first source is a wristband.
6. The system of paragraph 1, where the first source is one of the group of first sources, where the group of first sources consists of: a bar code, a bar code reader, a wristband, a tag, a drug label, laser readable data, and radio-frequency readable data.
7. The system of paragraph 1, where the second computer is a personal digital assistant.
8. The system of paragraph 1, where the second source is one of a group of second sources, where the group of second sources consists of: a medication label, a bar code, a bar code reader, a wristband, a tag, a drug label, laser readable data, and radio-frequency readable data a bar code.
9. The system of paragraph 1, where the medication identifier is one of a group of medication identifiers, where the group of medical identifiers consists of: a drug name, a dosage, a manufacturer, a batch, an expiration date, a National Drug Code (NDC) number, a proprietary database drug identifier, a company product code number, and a drug prescriber.
10. The system of paragraph 1, where the first computer is designed to send the operating parameter to the infusion pump if the second patient identifier and the third patient identifier are equivalent to the first patient identifier.

## Claims

1. A method for operating a medical device comprising the steps of: storing medical treatment data in a memory associated with a first processor, the medical treatment data including a first patient identification data, a first medication identification data, and a first plurality of medical device operating parameters, where the first plurality of medical device operating parameters is associated with the medical treatment data and the patient identification data; inputting second medication identification data into a second processor, where the second medication identification data is associated with medication to be administered to a patient, where the medical device is operably connected to the second processor; inputting second patient identification data into the second processor; sending the second medication identification data and the second patient identification data from the second processor to the first processor; finding a latest plurality of medical device operating parameters in the memory associated with the first processor, and sending the latest plurality of medical device operating parameters to the second processor if a comparison of the first and second patient identifiers satisfies a first predetermined condition, and if a comparison of the first and second medication identification data satisfies a second predetermined condition; sending a confirmation to the second processor if the first plurality of operating parameters is equivalent to the latest plurality of operating parameters; sending the latest plurality of operating parameters to the medical device if the first plurality of operating parameters is equivalent to the latest plurality of operating parameters.

2. The method of Claim 1, further comprising the step of inputting into the first processor a second medication identifier, where the step of sending the latest plurality of operating parameters to the medical device is performed only if the first and second medication identifiers are equivalent.

3. The method of Claim 1, where the first source is a wristband.

4. The method of Claim 1, where the second computer is at a remote location.

5. The method of Claim 1, where the second processor is the processor of a digital assistant.

6. The method of Claim 1, where the second source is a medication label.

7. The method of Claim 1, further comprising the step of using the operating parameter to program the medical device.

8. A program for operating a medical device, the program stored on a computer readable medium, the program comprising logic for: storing medical treatment data in a memory associated with a first processor, the medical treatment data including a first patient identification data, a first medication identification data, and a first plurality of medical device operating parameters, where the first plurality of medical device operating parameters is associated with the medical treatment data and the patient identification data; accepting a second medication identification data into a second processor, where the second medication identification data is associated with medication to be administered to a patient, where the medical device is operably connected to the second processor; accepting a second patient identification data into the second processor; sending the second medication identification data and the second patient identification data from the second processor to the first processor; finding a latest plurality of medical device operating parameters in the memory associated with the first processor; sending the latest plurality of medical device operating parameters to the second processor if a comparison of the first and second patient identifiers satisfies a first predetermined condition, and if a comparison of the first and second medication identification data satisfies a second predetermined condition; sending a confirmation to the second processor if the first plurality of operating parameters is equivalent to the latest plurality of operating parameters; and sending the latest plurality of operating parameters to the medical device if the first plurality of operating parameters is equivalent to the latest plurality of operating parameters.

9. The program of Claim 8, further comprising logic for accepting into the first processor a second medication identifier, where the step of sending the latest plurality of operating parameters to the medical device is performed only if the first and second medication identifiers are equivalent.

10. The program of Claim 8, where the first source is a wristband.

11. The program of Claim 8, where the second computer is at a remote location.

12. The program of Claim 8, where the second processor is the processor of a digital assistant.

13. The program of Claim 8, where the second source is a medication label.

14. The program of Claim 8, further comprising logic for programming the medical device using the latest plurality of operating parameters.
